# EUROPEAN PATENT APPLICATION

(11) **EP 3 438 985 A1**
(43) Date of publication of application: **06.02.2019**
(21) Application number: 18186614.6
(22) Date of filing: 31.07.2018
(51) Int. Cl.: G16H 10/60, G16H 10/40

(54) **HEALTH STATUS MATCHING SYSTEM AND METHOD**

(30) Priority: 31.07.2017 US 201715664639
(71) Applicant: Michael, Azeem, Cortlandt Manor NY 10567 (US)
(72) Inventor: Michael, Azeem, Cortlandt Manor NY 10567 (US)
(74) Representative: Linhart, Friedrich Karl Eberhard

(57) **Abstract**

Techniques are disclosed for encrypting a user's health status and matching a user's profile with another user's profile based on each user's preferences for their potential partner's health status and the users' health statuses. Each user can request his or her health care provider to verify his or her medical information to ensure that his or her health status is up to date. Upon receiving verification from the user's health care provider, a match making application encrypts the user's health status by generating a code that corresponds to the user's health status. The application analyzes the user's preferences for his or her potential partner's health status and the potential partner's health status in order to generate a match. After determining a match result, the match making application generates a matching code that corresponds with a specific color for display on an application user interface on user devices.

## Description

### FIELD OF THE INVENTION

The present invention relates generally to a system and method for processing and managing health care related information. More particularly, the present invention is directed to a system and method for sharing medical information without divulging actual medical data.

### BACKGROUND OF THE INVENTION

Getting tested for sexually transmitted diseases (STDs) and screening potential partners is the only way for a sexually active person to be sure of their STD status and stop the spread of STDs among their sexual partners. However, sharing information about STD status and other health information can cause embarrassment and awkwardness among partners. In this way, many individuals are reluctant to openly communicate with their partners about their health status. Thus, there is a need in the prior art for individuals to share their medical information without sharing the actual medical data. In this regard, the invention described herein addresses this problem.

### PROBLEM TO BE SOLVED

It is the object of the invention to solve or to at least diminish the above-mentioned disadvantages.

### SOLUTION TO THE PROBLEM

The problem is solved by a computer-implemented method for displaying on a mobile device health status matches between two or more users, comprising the steps of: receiving health information associated with a first user via an application user interface of a match making application, the health information comprising health status of the first user and being received from the first user via a first user device; receiving health criteria for a potential partner from a second user via the application user interface, the health criteria for the potential partner being received from the second user via a second user device; generating a health status code associated with the health information of the first user; determining whether the health status of the first user match the health criteria for the potential partner received from the second user; and generating a matching code that corresponds with a color for display on the application user interface on the first user device and the second user device.

In a typical embodiment, the health information comprises user's STD test results.

In a typical embodiment, the method further comprises the steps of: receiving a request from the first user for verification of the health information of the first user; generating an access token to transmit to a provider terminal, the provider terminal being operated by a health care provider; receiving verification of the health information of the first user the verification being received from the health care provider having an access to a patient medical information database comprising the health information of the first user.

In a typical embodiment, the method further comprises the steps of: receiving, from the first user device, a request to connect the first user device and the second user device; generating a one-time access code to transmit to the second user device; authenticating and authorizing the second user device to determine whether the health status of the first user matches the health criteria for the potential partner received from the second user.

In a typical embodiment, the method further comprises the steps of: receiving, from the first user device, a request to connect the first user device and the second user device; generating a QR code to display on the application user interface at the first user device; authenticating and authorizing the second user device to determine whether the health status of the first user matches the health criteria for the potential partner received from the second user.

In a typical embodiment, the method further comprises the steps of: receiving, from the first user device, a request to connect the first user device and the second user device; displaying a profile picture of the second user on the application user interface at the first user device; authenticating and authorizing the second user device to determine whether the health status of the first user matches the health criteria for the potential partner received from the second user.

In a typical embodiment, the method further comprises the steps of: displaying a prompt to schedule a doctor's appointment on the application user interface; receiving a request to book the doctor's appointment; retrieving available dates and times for the doctor's appointment, the available dates and times being received from a health care provider at a provider terminal; booking the doctor's appointment based at least in part on the selection of one of the available dates and times.

In a typical embodiment, the color is green if the health status of the first user matches the health criteria for the potential partner received from the second user and the health status of the first user is current.

In a typical embodiment, the color is yellow if the health status of the first user matches the health criteria for the potential partner received from the second user and the health status of the first user is out of date.

In a typical embodiment, the color is red if the health status of the first user does not match the health criteria for the potential partner received from the second user.

A network computer system for displaying health status matches between two or more users comprises the following: a memory unit for storing instructions and a processor that executes said instructions to enable actions, including: receiving health information associated with a first user via an application user interface of a match making application, the health information comprising health status of the first user and being received from the first user via a first user device; receiving health criteria for a potential partner from a second user via the application user interface, the health criteria for the potential partner being received from the second user via a second user device; generating a health status code associated with the health information of the first user; determining whether the health status of the first user match the health criteria for the potential partner received from the second user; and generating a matching code that corresponds with a color for display on the application user interface on the first user device and the second user device.

In a typical embodiment, said health information comprises user's STD test results.

In a typical embodiment, the system further comprises the steps of: receiving a request from the first user for verification of the health information of the first user; generating an access token to transmit to a provider terminal, the provider terminal being operated by a health care provider; receiving verification of the health information of the first user the verification being received from the health care provider having an access to a patient medical information database comprising the health information of the first user.

In a typical embodiment, the system further comprises the steps of: receiving, from the first user device, a request to connect the first user device and the second user device; generating a one-time access code to transmit to the second user device; authenticating and authorizing the second user device to determine whether the health status of the first user matches the health criteria for the potential partner received from the second user.

In a typical embodiment, the system further comprises the steps of: receiving, from the first user device, a request to connect the first user device and the second user device; generating a QR code to display on the application user interface at the first user device; authenticating and authorizing the second user device to determine whether the health status of the first user matches the health criteria for the potential partner received from the second user.

In a typical embodiment, the system further comprises the steps of: receiving, from the first user device, a request to connect the first user device and the second user device; displaying a profile picture of the second user on the application user interface at the first user device; authenticating and authorizing the second user device to determine whether the health status of the first user matches the health criteria for the potential partner received from the second user.

In a typical embodiment, the system further comprises the steps of: displaying a prompt to schedule a doctor's appointment on the application user interface; receiving a request to book the doctor's appointment; retrieving available dates and times for the doctor's appointment, the available dates and times being received from a health care provider at a provider terminal; booking the doctor's appointment based at least in part on the selection of one of the available dates and times.

In a typical embodiment, the color is green if the health status of the first user matches the health criteria for the potential partner received from the second user and the health status of the first user is current.

In a typical embodiment, the color is yellow if the health status of the first user matches the health criteria for the potential partner received from the second user and the health status of the first user is out of date.

In a typical embodiment, the color is red if the health status of the first user does not match the health criteria for the potential partner received from the second user.

### BRIEF DESCRIPTION OF THE DRAWINGS

**FIG. 1** shows an exemplary architecture for connecting two or more users based on the users' health status preferences and health statuses.
**FIG. 2** is a block diagram showing various components of one or more illustrative computing devices that implement users' health status preferences and health statuses matching.
**FIG. 3** is an exemplary work flow of requesting and receiving a verification for a user's medical information from a provider terminal and establishing a connection between user devices to determine a match.
**FIG. 4** is a flow diagram of an example process for setting up a user profile and receiving a verification of a user's medical information from a provider.
**FIG. 5** is a flow diagram of an example process for specifying a user's health status preferences.
**FIGs. 6** through **8** are flow diagrams of example processes for establishing a connection between user devices and receiving authentication and authorization in order to determine a match.
**FIG. 9** is a flow diagram of an example process for matching health status preferences and health statuses for two or more users.

### DETAILED DESCRIPTION OF THE INVENTION

Techniques are disclosed herein for encrypting a user's health status and matching a user's profile with another user's profile based on each user's preferences for their potential sexual partner's health status and the users' health statuses. Some embodiments of the techniques include creating a user's profile, requesting verification of a user's health information from a provider, providing an access token to authenticate and authorize the provider to access and review the user's health information, and importing, to the user's profile, the verified medical information from the provider. The techniques further include identifying a partner's user device, requesting a connection to match the user's profile with the partner's profile based on their preferences for each other's health status and the users' health statuses, receiving a consent to connect, generating a code to display their match status, and displaying their match status on each of the user device and the partner's user device.

In various embodiments, the present system comprises a match making application having a user account manager for managing a user profile, a health care provider profile, and user medical data, a profile matching module for filtering a user's health status preference criteria for a potential partner and analyzing the users' health status preference criteria and the potential partner's health status, and a security and protocol management for authenticating and authorizing access to a user's health information and generating access tokens and/or codes. The application is accessible via network enabled devices and supported via one or more application servers in the network, wherein the servers can comprise a computer system having a memory unit with instructions stored thereon, and a processor that is configured to execute the instructions, resulting in the application for enabling a user to connect with one or more other users (i.e., potential sexual partners) for determining whether their health statuses meet each other's health status preference criteria.

The application is configured to allow the user to automatically import from a data source his or her health information or manually input his or her health information (e.g., date of last STD screening, STD screening results) and set one or more filters that define the user's health status preference criteria for a potential partner via an application user interface. In some embodiments, the filters include, without limitation, the potential partner's test results, the number of other partners the potential partner has had (other than the user) since the partner's last checkup (i.e., doctor's appointment), and the amount of time since the last checkup, and/or so forth.

Based on the user's health status preference criteria and the potential partner's health status or information, the application is configured to determine whether the user's health status preference criteria and a potential partner's health status is a match or a not a match, and vice versa. Some embodiments of the application provide an application user interface having a graphic user interface (GUI) for displaying match results in an encrypted manner, thereby hiding any protected health information and other private information. Preferably, the GUI includes colored screens and/or icons, wherein each of the colors represents different match and/or non-match combinations. For example, a green screen or icon signifies that the user's health status preference criteria and the potential partner's health status are a match. Similarly, a yellow screen or icon signifies that the user's health status preference criteria and the potential partner's health status are a match, but one or both of the user's health information and/or the potential partner's health information is outdated. Optionally, the green screen and yellow screen may comprise additional annotations for showing that one or both of the user's health information and/or the potential partner's health information has been verified or unverified by a health care provider. Finally, a red screen or icon signifies that the user's health status preference criteria and the potential partner's health status are not a match.

In various embodiments, the application generates a token or an encrypted code, wherein the token or the code corresponds to the user's health status, further wherein the token or the code is used to determine a match between two or more users. Once the token or the code is used to determine a match, the token or the code expires so that the user's health status is not saved. In this regard, the techniques disclosed herein safeguard the user's actual medical data while enabling the user to share the user's health status to prevent spreading of diseases.

In various embodiments, the application can authenticate and authorize a first user device operated by a user and a second user device operated by a potential partner of the first user before determining whether the user's health status preference criteria and the partner's health status is a match or a not a match, and vice versa. As used herein, the terms "second user" and the "potential partner" or "the potential partner of the first user" can be used interchangeably unless the context clearly suggests otherwise. Additionally, the first user can be the potential partner of the second user and the order does not matter as the terms "first" and "second" are used to disclose the concept of the invention in a simplified and more concrete form. The first user can send the second user an invitation to match by text message, email, or in-app messaging, wherein the content of the text message, email, or in-app messaging can comprise a one-time use code or uniform resource locator (URL) that can be activated to give consent in order to determine whether their health status preference criteria and health status match or does not match. Some embodiments may further comprise means for authenticating and authorizing user devices via near field communication (NFC) such that users in close proximity can easily give consent and share information.

Some embodiments of the application enable a user to request verification of his or her health information from a health care provider. In this regard, the application can automatically generate and transmit a message to the user's health care provider via an application programming interface (API), wherein the message comprises a one-time use URL or an access token that can be activated by the health care provider to view, enter, and/or verify the user's health information. If the user's health information is outdated, some embodiments of the application may be configured to enable the user to request and book a doctor's appointment with the health care provider.

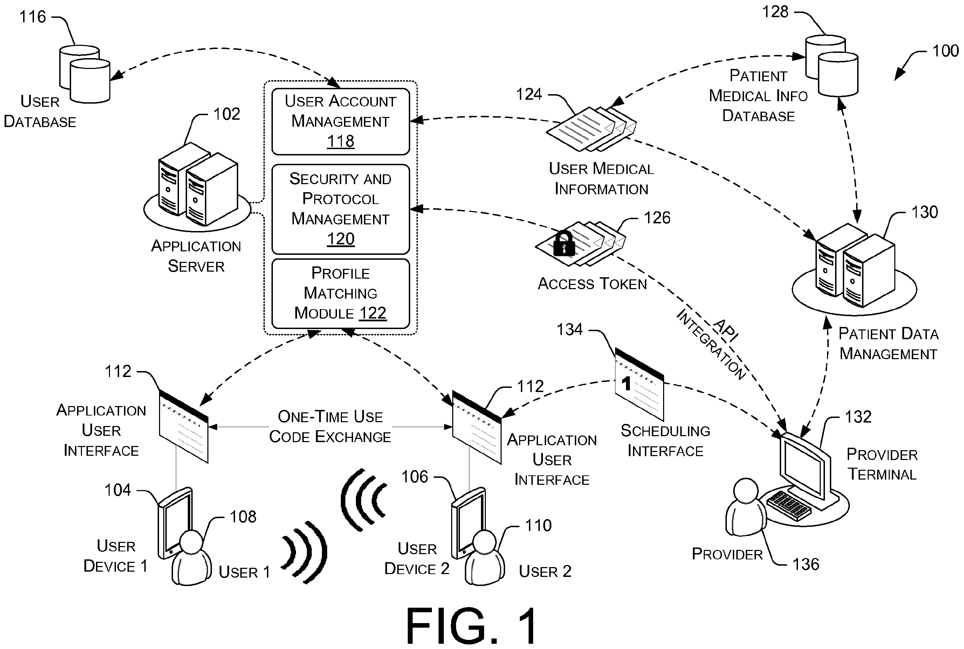

**FIG. 1** shows an exemplary architecture for connecting two or more users based on the users' health status preference criteria and health statuses. The system **100** comprises a first user device **104** operated by a first user **108** and a second user device **106** operated by a second user **110** or a potential partner, wherein the user devices **104, 106** are connected to a network (e.g., the Internet, LAN, etc.). The user devices **104**, **106** comprise various types of computer systems such as a mobile phone, a tablet computer, a personal digital assistant (PDA), an e-reader, and/or so forth. In the illustrated embodiment, the system and method of the present invention are taught and disclosed in terms of mobile computing. It should be understood, however, that the same principles are applicable to nearly any device capable of executing a machine-readable instruction.

The user devices **104, 106** can access a match making application, wherein the application comprises an application user interface **112** and can reside at least in part on the user devices **104**, **106**. In various embodiments, the match making application can comprise a mobile application, a web application, a website, a plug-in, and/or other types of downloadable and/or non-downloadable software program. The match making application can execute on one or more computing devices in the system **100**, such as an application server **102**. The application server **102** can be distributed processing computing devices that are scalable according to workload demand. The application server **102** can include general-purpose computers, such as desktop computers, tablet computers, laptop computers, servers, or other electronic devices that are capable of receiving inputs, processing the inputs, and generating output data. In still other embodiments, the one or more application servers **102** (i.e., computing devices) may be virtual computing devices in the form of computing devices, such as virtual machines and software containers. The application server **102** may store data in a distributed storage system, in which data may be stored for long periods of time and replicated to guarantee reliability. Accordingly, the application server **102** may provide data and processing redundancy, in which data processing and data storage may be scaled in response to demand. Further, in a networked deployment, new application servers **102** may be added without affecting the operational integrity of the application.

The application comprises a user account management **118**, a security and protocol management **120**, and a profile matching module **122**. The user account management **118** can manage user profiles and user medical data associated with each of the users **108**, **110**. In this regard, the user account management **118** receives and collects, from the user devices **104**, **106,** data related to user profiles and the user's medical information. User profile information can include user name, age, sex, user's contact information, user's health criteria for potential partners, user preferences, user's health care provider information, profile pictures, and/or so forth. Additionally, user profile information can include user login information, such as user identification and password, user credentials, account settings, payment information, and/or so forth.

The user account management **118** also receives and collects, from the user devices **104**, **106** and/or one or more data sources, medical information relating to each of the users **108**, **110**. The user medical information **124** can comprise test or screening results (e.g., STD screening information) and clinical data, patient medical history, medication, treatment and/or procedures, surgeries, medical status, diseases, diagnosis and illnesses, family medical history, and/or so forth. Thus, the user medical information **124** comprises the user's health status. The user medical information **124** can also include health care providers' (e.g., primary care physician) information such as name and contact information, pharmacy information, medical insurance information, and/or so forth. The medical information can be derived from a user database **116** that collects, stores, and maintains information related to each user **108**, **110.** The user database **116** can receive and collect information from the users **108**, **110** via the user devices **104**, **106** and/or third parties (i.e., non-users). The user medical information **124** can also be derived from a patient medical information database **128** of a patient data management **130** that can be associated with and/or managed by, for example, a hospital, clinic, patient services, and/or other medical facilities. The databases may store data across multiple virtual data storage clusters with redundancy, so that the data may be optimized for quick access. For example, user database **116** and the patient medical information database **128** can also be partially replicated on the user device. The patient data management **130** can communicate with a provider terminal **132** that is operated by a health care provider **136** (i.e., the user's primary care physician) so as to allow the health care provider **136** to retrieve a user's medical information upon receiving a request from the user for verifying the user's medical information and/or for requesting the user's medical information and/or records.

To establish authenticate and authorize health care providers **136** to access, review, and verify a user's medical information, the security and protocol management **120**, upon receiving a request from the user device **104**, **106**, can generate and send an access token **126**, a secure link (e.g., a URL), and/or a code to the provider terminal **132** via, for example, an API. The security and protocol management **120** can transmit the access token **126** in a message or an email to the health care provider **136** at the provider terminal **132**. The access token **126** can be configured to expire after a predetermined period of time. Upon activating the access token **126**, the health care provider **136** can be prompted to create a provider account in order to view, enter, and/or confirm the user's medical information, including the user's STD screening information. Alternatively, the provider **136** can proceed as a guest to view, enter, and/or confirm the user's medical information. In various embodiments, the provider **136** can be prompted to further verify his or her identification or credentialing information (e.g., license number) to ensure that the provider is a qualified medical professional to view, enter, verify, and/or confirm the user's health information.

In various embodiments, the security and protocol management **120** can bypass the health care provider **136** in order to obtain the user's medical information directly from the patient medical information database **128** of the patient data management **130.** In this regard, the security and protocol management **120** allows the user device and the patient data management **130** to conduct a protocol handshake in order to authenticate and authorize the patient data management **130** to retrieve and export the user's medical information **124** to the user profile. In various embodiments, the patient data management **120** may use data adaptors to retrieve data from the structured or unstructured databases of the patient medical information database **128.** Additionally, the patient data management **120** may include a workflow scheduler that periodically checks for and retrieves newly available data from the patient medical information database **128.** The workflow scheduler may handle the extraction and the handling of the data based on configurable policies. For example, a configurable policy may specify the source data location, frequency of data retrieval, data retention period, and data disposal following an expiration of the data retention period.

In various embodiments, users can also request an appointment for a checkup with their health care provider **136** via a scheduling interface **134.** The application, via the scheduling interface **134** can also alert users if their medical information is outdated and/or if more than a predetermined amount of time has passed since their last doctor's appointment. In various embodiments, the application can automatically prompt the users to schedule a checkup if their medical information is outdated and/or if more than a predetermined amount of time has passed since their last doctor's appointment. More specifically, the scheduling interface **134** may be configured to access the health care provider's calendar to determine the health care provider's availability (e.g., date and time) and display on the application user interface **112** the health care provider's availability for an appointment. In various embodiments, the scheduling interface **134** is also configured to access the user's calendar via the user device in order to determine the user's availability and correlate the user's availability and the health care provider's availability to display on the application user interface **112** the date and time when both the user and the health care provider are available for an appointment. Based on the availabilities of the health care provider and/or the user, the user can select a desired appointment time on the scheduling interface **134** in order to schedule a checkup. In various embodiments, the scheduling interface **134** can integrate with the user's calendar in order to block time on the user's calendar and display on the user's calendar the appointment time. Additionally, the scheduling interface **134** can send reminders to the user for any upcoming appointments.

Two or more devices **104**, **106** can establish communication by sending and receiving a one-time use code. In various embodiments, the devices **104**, **106** can connect via NFC in close proximity. When a first user device **104** recognizes a second user device **106**, the application user interface **112** displays on the first user device **104** the second user's or the potential partner's **110** profile picture, name, or other identifier so as to allow the first user **108** to confirm connection with the correct user or potential partner **110.** The application user interface **112** can also display a set of buttons on which the first user **108** can click or tap to confirm that the displayed profile picture, name, or other identifier belongs to the correct user or potential partner. For example, the buttons can comprise a check mark for confirming that it is the correct user or potential partner or an x mark to indicate that it is not the correct user or potential partner. If the first user device **104** does not detect the correct second user, and the first user indicates that the correct second user is not found, the first user device **104** searches again for the correct second user.

In various embodiments, upon confirming that the first user **108** is connecting with the correct user or potential partner **110**, the first user device **104** transmits to the second user device **106** a one-time use code. The one-time use code can also be transmitted from the first user device **104** to the second user device **106** remotely by entering the second user's user name or identifier on the application user interface **112** from the first user device **104.** Thus, if the first user knows the second user's user name or another identifier, the first user can enter the second user's user name or another identifier in the application user interface **112** from the first user device **104** in order to locate the second user and transmit the one-time use code. The one-time use code can be transmitted in a message (e.g., SMS, email, in-app messaging, etc.). The one-time use code is unique to the first user device **104** and can expire after a predetermined period of time. In various embodiments, the one-time use code can comprise a link that can be activated in order to authenticate and authorize the other user device. Additionally, the one-time use code can be entered on the application user interface **112** in order to authenticate and authorize the other user device.

In various embodiments the devices **104**, **106** can connect via quick response (QR) codes. In this regard, the first user device **104** can generate a QR code for display on the application user interface **112.** The second user device **106** can scan the QR code displayed on the first user device **104** via an image capturing device (e.g., a camera) on the second user device **106** in order to authenticate and authorize the other user device.

Upon authenticating and authorizing the user devices **104**, **106** to conduct a match making analysis, the profile matching module **122** is configured to encrypt a user's health status into a code or a token and determine whether the user's health status matches the other user's health status preferences for a potential partner. Each user's health status preferences for a potential partner is set by selecting various health criteria that the user seeks in a potential sexual partner, wherein the health criteria can be selected by filters on the application user interface **112** from each of the respective user devices **104**, **106**. In one embodiment, the health criteria include the number of partners since the potential partner's last checkup, the number of months since the last checkup, and the test results of various STD screening. The user's health status in an encrypted format or code is used to determine whether the user's health status matches the potential sexual partner's health status preferences and vice versa. The encrypted format of the health status or the health status code can be set to expire after a predetermined amount of time and/or after the match making analysis so that the user's health status information is not saved, archived, and/or stored.

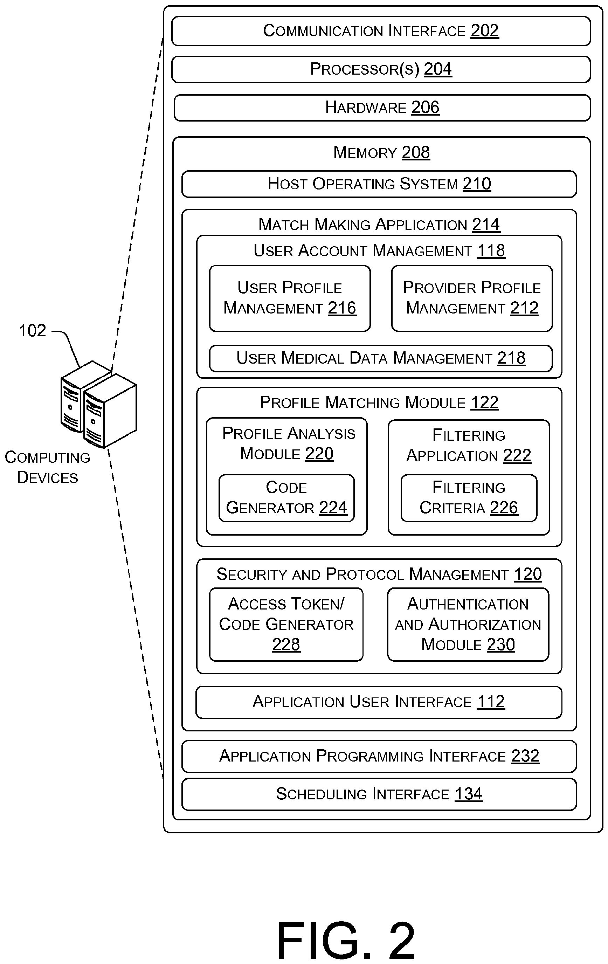

**FIG. 2** is a block diagram showing various components of one or more illustrative computing devices that implement health status preference matching. The number of computing devices **102** may be scaled up and down by a distributed processing control algorithm based on the data processing demands of the application, data store, and/or other components in the system. For example, during peak performance data processing times, the number of computing devices **102** that are executing match making analysis functionalities may be scaled up on the fly based on processing demand. However, once the processing demand drops, the number of computing devices **102** that are executing the match making analysis functionalities may be reduced on the fly. Such scaling up and scaling down of the number of computing devices **102** may be repeated based on processing demand. The computing devices **102** may include a communication interface **202**, one or more processors **204**, hardware **206**, and a memory unit **208.** The communication interface **202** may include wireless and/or wired communication components that enable the devices to transmit data to and receive data from other networked devices. The hardware **206** may include additional hardware interface, data communication, or data storage hardware. For example, the hardware interfaces may include a data output device (e.g., visual display, audio speakers), and one or more data input devices. The data input devices may include but are not limited to, combinations of one or more of keypads, keyboards, mouse devices, touch screens that accept gestures, microphones, voice or speech recognition devices, cameras, and any other suitable devices.

The memory unit **208** may be implemented using computer-readable media, such as computer storage media. Computer-readable media includes, at least, two types of computer-readable media, namely computer storage media and communications media. Computer storage media includes volatile and non-volatile, removable and non-removable media implemented in any method or technology for storage of information such as computer-readable instructions, data structures, program modules, code segments, or other data. Computer storage media includes, but is not limited to, RAM, ROM, EEPROM, flash memory or other memory technology, CD-ROM, digital versatile disks (DVD), high-definition multimedia/data storage disks, or other optical storage, magnetic cassettes, magnetic tape, magnetic disk storage or other magnetic storage devices, or any other non-transmission medium that can be used to store information for access by a computing device. In contrast, communication media may embody computer-readable instructions, data structures, program modules, code segments, or other data in a modulated data signal, such as a carrier wave, or another transmission mechanism.

The processors **204** and the memory unit **208** of the computing devices **102** may implement an operating system **210.** In turn, the operating system **210** may provide an execution environment for the match making application **214**, the application programming interface **232**, and the scheduling interface **134**. The operating system **210** may include components that enable the computing devices **102** to receive and transmit data via various interfaces (e.g., user controls, communication interface, and/or memory input/output devices), as well as process data using the processors **204** to generate output. The operating system **210** may include a presentation component that presents the output (e.g., display the data on an electronic display, store the data in memory, transmit the data to another electronic device, etc.). Additionally, the operating system **210** may include other components that perform various additional functions generally associated with an operating system.

The match making application **214** comprises the user account management **118**, the profile matching module **122**, the security and protocol management module **120**, and the application user interface **112.** The user account management **118** comprises a user profile management module **216**, a provider profile management module **212**, and a user medical data management module **218.** The user profile management module **216** collects and maintains information and data related to a user profile associated with a user. The user profile management module **216** can collect information directly from a user via a user device and/or from other data sources (e.g., the user's social media account). User profile information includes user name, age, sex, user's contact information, user's health criteria for potential partners, user preferences, user's health care provider information, profile pictures, and/or so forth. Additionally, user profile information can include user login information, such as user identification and password, user credentials, account settings, payment information, and/or so forth.

The user medical data management **218** collects and maintains information and data related to the user medical or health information. The user medical information can comprise test or screening results such as STD screening information and clinical data, patient medical history, medication, treatment and/or procedures, surgeries, medical status, diseases, diagnosis and illnesses, family medical history, and/or so forth. The user medical data management **218** can collect user medical or health information directly from a user via a user device, from the user's health care provider via a provider terminal, and/or other data sources such as the patient medical information database and the patient data management.

The provider profile management module **212** collects and maintains information and data related to a user's health care provider (e.g., primary care physician). The provider profile management module **212** can collect health care provider information from a health care provider via a provider terminal, a user via a user device, and/or other data sources. The health care provider information can comprise the health care provider's name, contact information, hospital or clinic information, areas of specialty, affiliation, the health care provider's credentials, network and out-of-network information, and/or so forth.

The user account management **118** may use data adaptors to retrieve data from the structured or unstructured databases of the data sources described above (e.g., patient medical information database). Because the structured databases can provide data that are accessible via simple data retrieval algorithms, the data collection module can use data-agnostic data adaptors to access the data sources without taking into consideration the underlying content of the data. Further, changes to the data content in each data source do not affect the functionality of the corresponding data-agnostic data adaptors. Alternatively, the user account manager **118** may use database-specific data adaptors to access structured databases.

In some embodiments, the user account manager **118** may include a workflow scheduler that periodically checks for and retrieves newly available data from the multiple data sources. The workflow scheduler may handle the extraction and the handling of the data based on configurable policies. For example, a configurable policy may specify the source data location, frequency of data retrieval, data retention period, and data disposal following an expiration of the data retention period. The application user interface **112** can comprise a GUI for the updating a user's profile information, health criteria for potential partners, and/or other settings. In some embodiments, the application user interface **112** comprises a home page that includes an option for viewing previous match making results, a countdown of the number of days until next doctor's appointment, a link to make a doctor's appointment, and a link to find a match.

The profile matching module **122** comprises a profile analysis module **220** and a filtering application **222.** The profile analysis module **220** is configured to analyze a user's health criteria for potential partners and a potential partner's health status to determine whether the potential partner's health status meets the user's health criteria for potential partners. In this regard, the profile analysis module **220** may implement adaptor-specific logics to decode the format of various data from respective data sources. In various embodiments, the profile analysis module **220** comprises a code generator **224** that encrypts the potential partner's health status by generating a code that represents the health status and uses the code to determine whether the potential partner's health status meets the user's health criteria for potential partners. The code generator **224** is further configured to generate a code based on the user and the potential partner's match status or result. In various embodiments, the code generator **224** is configured to generate a code that displays a colored screen or icon on the application user interface **112.** For example, the code generator **224** generates a code that displays a red screen on the application user interface **112** if the user's health criteria for potential partners and the potential partner's health status does not match. In another example, the code generator **224** generates a code that displays a green screen on the application user interface **112** if the user's health criteria for potential partners and the potential partner's health status match.

The filtering application **222** enables the user to select health criteria for potential partners on the application user interface **112.** The filtering application **222** provides filtering criteria **226**, wherein the filtering criteria **226** comprises the number of partners since the potential partner's last checkup, the number of months since the last checkup, and the test results of different STD screening. The filtering criteria **226** can be presented on the application user interface **112** to allow the user to specify the user's preferences. For example, the filtering criteria **226** can be selected using drop-down menus, check boxes, multiple-choice questions, fill in the blank boxes, and/or so forth. In various embodiments, the user can specify the number of partners since the potential partner's last checkup and the number of months since the last checkup using "+" and "-" buttons for increasing or decreasing numerical values that correspond to the filtering criteria **226.**

The security and protocol management **120** comprises access token/code generator **228** and an authentication and authorization module **230.** The access token/code generator **228** is configured to generate an access token to transmit to a provider terminal operated by a health care provider in order to permit the health care provider to access, review, and verify a user's medical information. In various embodiments, the application programming interface **232** is used to request the health care provider to access, review, and verify the user's medical information. The access token/code generator **228** further provides a one-time use access code to transmit to a user device in order to connect two or more users together to conduct match making. The access code is uniquely generated and can be set to expire after a predetermined period of time after it is generated. The access code can be transmitted to a user device in a message and/or displayed on the application user interface to enable the other user to type in the access code. The access token/code generator **228** is further configured to generate a QR code that can be displayed on the application user interface **112**, wherein the QR code can be scanned.

The authentication and authorization module **230** is configured to conduct a protocol handshake and to enable the first user device to authorize the potential partner device and vice versa to determine whether the potential partner's health status match the user's health criteria for potential partners and whether the user's health status match the potential partner's health criteria. It is noted that various handshake protocol of a secure communications standard (e.g., secure sockets layer (SSL), transport layer security (TLS)) can be used, depending upon embodiments. Once the protocol handshake is completed and the potential partner device receives authentication and authorization, the code generator **224** encrypts the potential partner's health status and the user's health status to determine whether the potential partner's health status meets the user's health criteria for potential partners and whether the user's health status meets the potential partner's health criteria via the profile analysis module **220.**

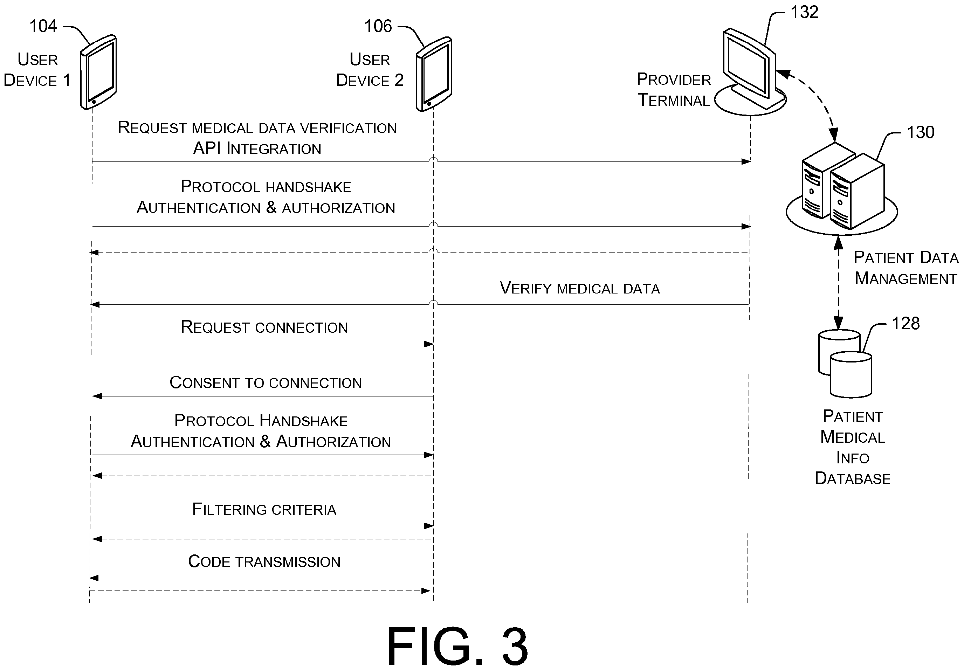

**FIGs. 3** through **9** present illustrative processes **300-900** for using the application to encrypt user health status and conduct match making analysis. The processes **300-900** are illustrated as a collection of blocks in a logical flow chart, which represents sequences of operations that can be implemented in hardware, software, or a combination thereof. In the context of software, the blocks represent computer-executable instructions that, when executed by one or more processors, perform the recited operations. Generally, computer-executable instructions may include routines, programs, objects, components, data structures, and the like that perform particular functions or implement particular abstract data types. The order in which the operations are described is not intended to be construed as a limitation, and any number of the described blocks can be combined in any order and/or in parallel to implement the process. For discussion purposes, the processes **300-900** are described with reference to **FIG. 1****.**

**FIG. 3** is an exemplary work flow of requesting and receiving a verification of a user's medical information from a patient data management and establishing a connection with a user device to determine a health status preference match. In the illustrated embodiment, a first user requests medical data verification from his or her health care provider at a provider terminal **132** via API using the first user device **104.** The first user device **104** can generate an access token that the provider terminal **132** receives from the first user device **104**, wherein the access token can be activated at the provider terminal **132** in order to authenticate and authorize the health care provider at the provider terminal **132.** The health care provider can obtain the first user's medical data from the patient medical information database **128** by requesting the patient data management **130** to retrieve the first user's medical data therefrom, wherein the patient data management **130** and the patient medical information database **128** are connected. Retrieved medical data is then verified by the health care provider at the provider terminal **132** and the first user's medical data can be marked "verified." In various embodiments, the medical data can also be imported to the first user's profile. This process can be repeated for the second user device **106** that is operated by a second user or a potential partner of the first user.

The first user device **104** can request to establish a connection with the second user device **106** when the two devices **104**, **106** are in close physical proximity (e.g., via NFC). Alternatively, the first user device **104** can transmit a one-time use code to the second user device **106** that the potential partner can activate at the second user device **106** in order to establish a connection with the first user device **104.** Further, the first user device **104** can generate a QR code that can be displayed on the application user interface at the first user device **104** that the second user device **106** can scan in order to establish a connection. Once the second user device **106** consents to a connection with the first user device **104** by confirming the identification of the first user operating the first user device **104**, entering a one-time use code, and/or scanning a QR code, the user devices **104**, **106** conduct a protocol handshake in order to conduct a match making analysis. The first user and the second user, via the first user device **104** and the second user device **106**, respectively, can exchange their health criteria for a potential partner and their health status in a code form to determine whether the second user's health status meets the first user's health criteria for a potential partner and the first user's health status meets the second user's health criteria for a potential partner.

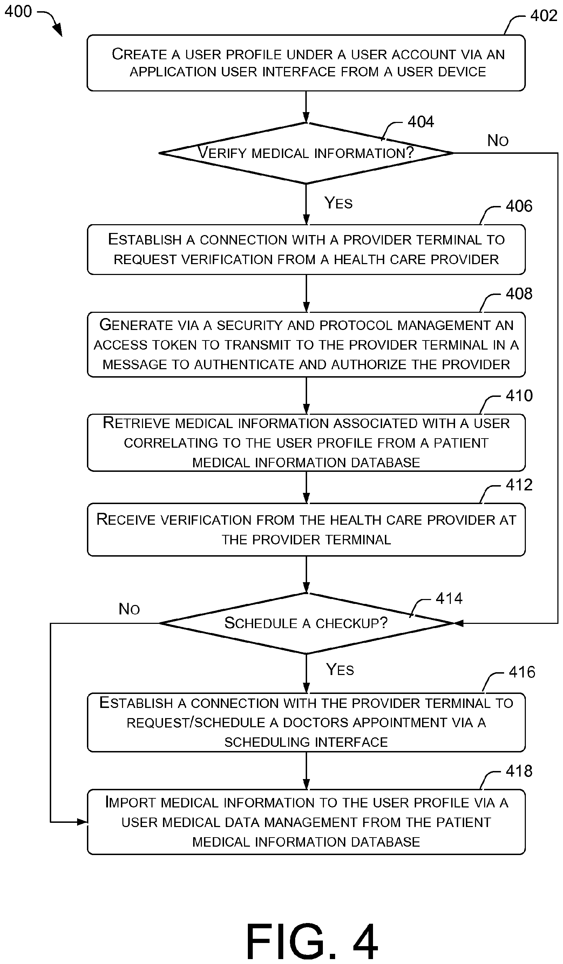

**FIG. 4** is a flow diagram of an example process **400** for setting up a user profile and receiving a verification of a user's medical information from a provider. As indicated in block **402**, a user account management of a match making application enables a user to create a user profile under a user account via an application user interface of the match making application from a user device. In various embodiments, the user can create a user account by linking the user's third party social networking accounts (e.g., Facebook™), in which case the user account management can obtain account related information from the third party social networking accounts. The user can set up his or her account by providing account related information such as name, location, age, gender, photo or an avatar, date of birth, and/or so forth. Account related information that is associated with each of the registered users is stored in a user database. The user can log into his or her account after creating a user account, for example, by providing his or her email address, user name, or other types of identifying information. After logging in, the user can set up a user profile by providing the user's health information related to the number of partners the user has had since last checkup or an appointment with a doctor or a health care provider, STD screening results, time passed since last checkup or appointment with a doctor or a health care provider, and/or so forth. At decision block **404**, if the user elects to have his or her health information verified by his or her health care provider ("yes" response from the decision block **404**), a security and protocol management of the match making application enables a user to establish a connection with a provider terminal from the user device to request verification from his or her health care provider as indicated in block **406.** In this regard, the application user interface can comprise a GUI that includes a button that can be activated by the user at the user device to request his or her health care provider to view, enter, and/or verify the user's health information.

Upon receiving a request to verify the user's health information, an access token/code generator of the security and protocol management automatically generates an access token to transmit to the provider terminal, as indicated in block **408.** The access token can be included in a message or an email to the health care provider (i.e., the user's primary care physician) of the user's choice using API. The token can comprise a link that can be activated. Once activated, the link can direct the health care provider to an application, a website, or a web page where the health care provider can create a provider account via the provider profile management in order to view, enter, verify, and/or confirm the user's STD screening information. In this regard, the health care provider may be prompted to provide verification or credentialing information to ensure that the provider is qualified to view, enter, verify, and/or confirm the user's health information. In various embodiments, the health care provider can proceed as a guest without creating a provider account in order to view, enter, verify, and/or confirm the user's health information.

At block **410**, the user medical data management allows the provider terminal to retrieve health information associated with the user correlating to the user profile from the patient medical information database. In various embodiments, the security and protocol management can bypass the health care provider at the provider terminal in order to extract the user's health information directly from the patient medical information database of the patient data management. Upon retrieving the health information associated with the user, the health care provider can review the information in order to verify the information. In various embodiments, the health care provider can include notes, memos, and/or comments as attachments the user's health information. Additionally, the health care provider can make corrections, amendments, and/or provide supplemental information. Upon verifying the user's health information, the health care provider can mark the health information as verified via the application user interface at the provider terminal. At block **412**, the user at the user device receives verification from the health care provider at the provider terminal.

At decision block **414**, the scheduling interface can prompt the user at the user device to schedule a checkup or a doctor's appointment with the health care provider. In some embodiments, the scheduling interface automatically prompts the user to schedule a checkup if more than a predetermined time has passed since the user's last doctor's appointment. For example, if more than twelve (12) months have passed since the user's last doctor's appointment, the scheduling interface can remind the user that he or she should make a doctor's appointment. Alternatively, the user can manually schedule a checkup without being prompted. Additionally, the user can still be prompted to schedule a checkup or schedule a checkup without a prompt if the user does not need his or her health care provider to verify his or her medical information ("no response from the decision block **404**). If the user elects to schedule an appointment ("yes" response from the decision block **414**), the scheduling interface establishes connection between the provider terminal and the user device to send a request for an appointment and to schedule the appointment based on the availability of the health care provider and/or the user as indicated in block **416.**

If the user does not need to schedule a checkup ("no" response from the decision block **414**), the user's medical information is imported to the user profile via the user medical data management from the patient medical information database as indicated in block **418.** Alternatively, the user can manually enter his or her medical information via the application user interface to specify the user's health status in his or her user profile. For instance, the user can enter the date that the user was last tested for STDs, and then indicate whether each of the corresponding tests yielded a positive or a negative result. For any unknown results, inconclusive results, or for tests that were not conducted, the user can indicate that the result is unknown. Thereafter, the user can confirm that the information is up to date and then update the information as needed.

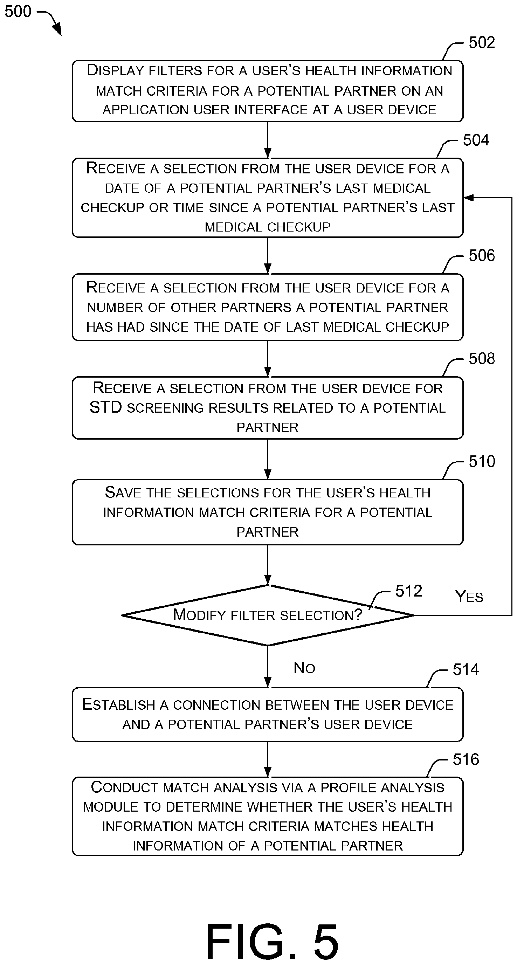

**FIG. 5** is a flow diagram of an example process **500** for specifying a user's health status preferences. As indicated in block **502**, the filtering application **222** displays filters for a user's health information match criteria for a potential partner on the application user interface. Said another way, the filters indicate the health criteria that the user seeks in a potential sexual partner (i.e., another user of the application). In an exemplary embodiment, the filters include the number of partners since the potential partner's last checkup, the number of months since the last checkup, and the test results of different STD screening. As indicated in block **504**, the filtering application receives a selection from the user device for a date of a potential partner's last medical checkup or time since a potential partner's last medical checkup. As indicated in block **506**, the filtering application receives a selection from the user device for a number of other partners a potential partner has had since the date of last medical checkup. As indicated in block **508**, the filtering application receives a selection from a user device for STD screening results related to a potential partner. As indicated in block **510**, the filtering application saves the selections for the user's health information match criteria for a potential partner. At decision block **512**, the user can modify the filtering selection via the filtering application. If the user elects to modify his or her filtering selections ("yes" response from the decision block **512**), the filtering application can receive a new selection for a date of a potential partner's last medical checkup or time since a potential partner's last medical checkup, a number of other partners a potential partner has had since the date of last medical checkup, and/or STD screening results related to a potential partner.

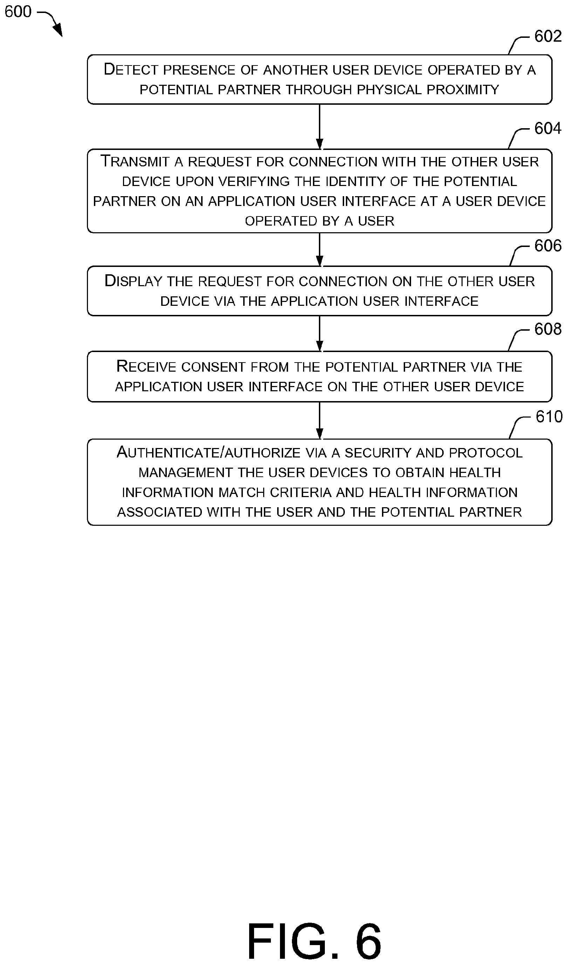

Upon saving the user's health status preferences for a potential partner, the user device establishes a connection with a potential partner's user device as indicated in block **514.** In various embodiments, the application is configured to request the user for consent to share the user's health status, and not the actual medical data, with another user. Detailed steps for establishing a connection between user devices and receiving authentication and authorization in order to transmit users' health information preferences and status are illustrated in **FIGs. 6** through **8****.** **FIG. 6** shows an example process **600** for connecting user devices via physical proximity using NFC. As indicated in block **602**, a first user device operated by a user can detect the presence of a second user device operated by a potential partner through physical proximity. Upon detecting the presence of the second user device, the application user interface displays on the first user device a profile picture of the potential partner in order to allow the user to verify that the first user device has detected the correct second user device. Upon verifying the correct potential partner, the first user device transmits a request for connection with the second user device as indicated in block **604.** As indicated in block **606**, the application user interface displays the request for connection on the second user device. As indicated in block **608**, the potential partner can enter consent via the application user interface on the second user device, which the first user device receives. As indicated in block **610**, the security and protocol management authenticates and authorizes the user devices to obtain health information match criteria and health information associated with the user and the potential partner.

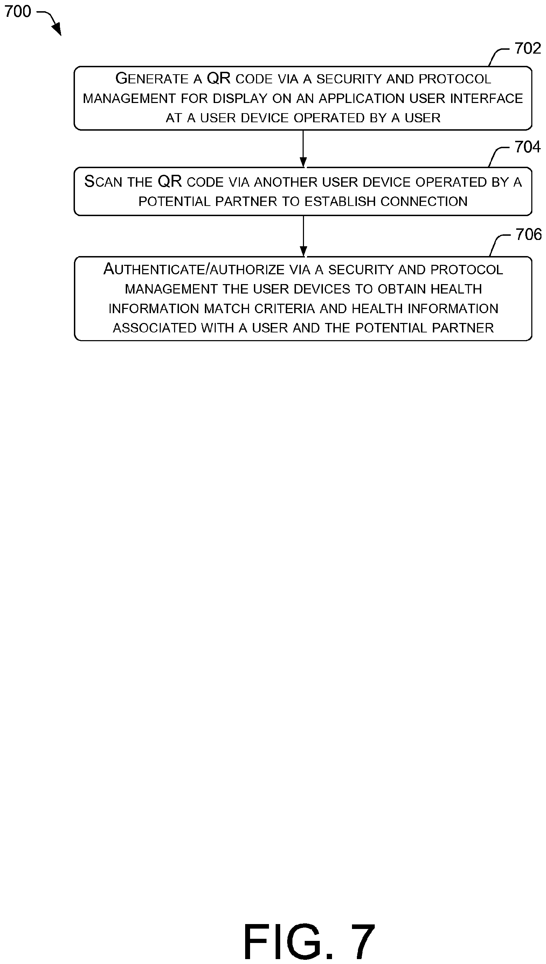

**FIG. 7** shows an example process **700** for connecting user devices using a QR code. As indicated in block **702**, the security and protocol management generates a QR code for display on the application user interface of a user device operated by a user. As indicated in block **704**, a second user device operated by a potential partner can scan the QR code to establish a connection between the user device and the second user device. Thus, it is contemplated that the application is configured to operate the second user device's camera to scan the QR codes. As indicated in block **706**, the security and protocol management authenticates and authorizes the user devices to obtain health information match criteria and health information associated with the user and the potential partner.

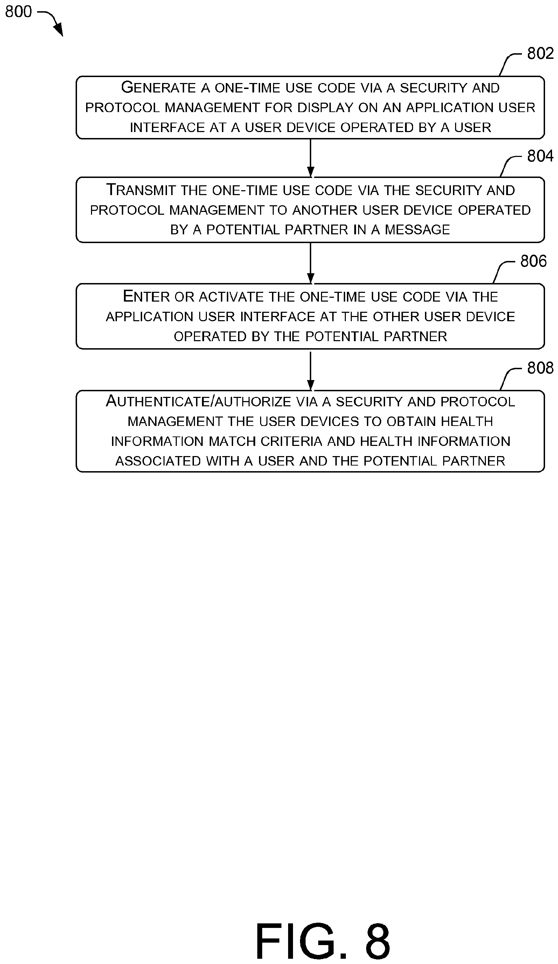

**FIG. 8** shows an example process **800** for connecting user devices using a one-time use code. As indicated in block **802**, the security and protocol management generates a one-time use code for display on the application user interface at a user device operated by a user. As indicated in block **804**, the security and protocol management transmits the one-time use code to a second user device operated by a potential partner. The one-time use code can be transmitted to the second user device in a message such as SMS, email, in-app message, and/or so forth. As indicated in block **806**, the potential partner can enter the one-time use code or activate the one-time use code via the application user interface at the second user device by the potential partner. As indicated in block **808**, the security and protocol management authenticates and authorizes the user devices to obtain health information match criteria and health information associated with the user and the potential partner.

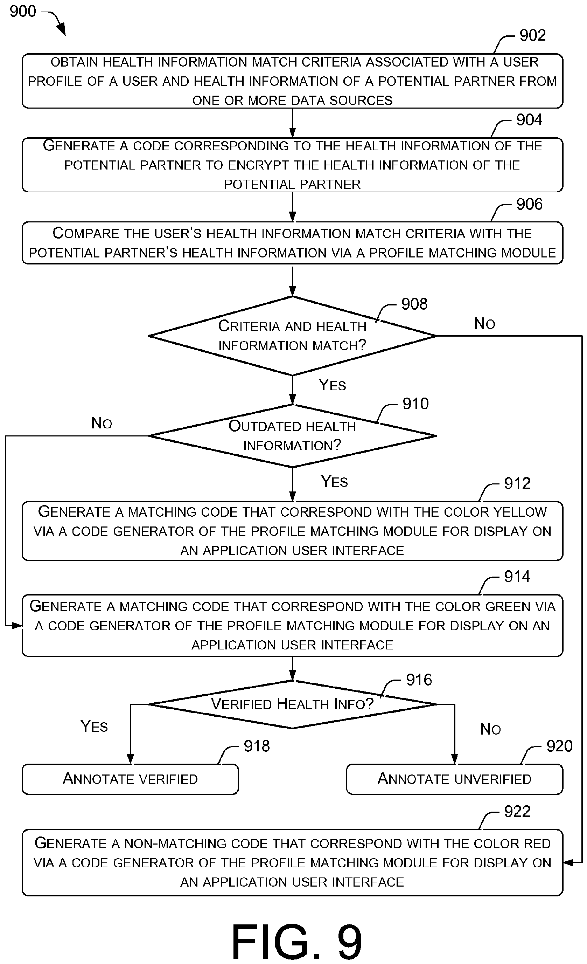

Referring back to **FIG. 5**, the profile analysis module conducts a match analysis in order to determine whether the user's health information match criteria meet health information of a potential partner as indicated in block **516.** Detailed steps for conducting a match analysis are shown in **FIG. 9. FIG. 9** is a flow diagram of an example process for matching health status preferences for two or more users. As indicated in block **902**, the profile matching module obtains health information match criteria associated with a user profile of a user and health information of a potential partner from one or more data sources. As indicated in block **904**, the code generator generates a code corresponding to the health information of the potential partner to encrypt the health status of the potential partner. As indicated in block **906**, the profile matching module compares the user's health information match criteria with the potential partner's health information. As indicated in decision block **908**, the profile matching module determines whether the user's health information match criteria and the potential partner's health information match. If there is a match ("yes" response from the decision block **908**), the scheduling interface determines whether the user's health information is outdated at decision block **910.**

If the user's health information is outdated (i.e., more than a predetermined amount of time has passed since the date of last screening or testing), the code generator of the profile matching module generates a matching code that correspond with the color yellow for display on an application user interface at the user devices as indicated in block **912.** If the user's health information is not outdated, the code generator of the profile matching module generates a matching code that corresponds with the color green for display on the application user interface as indicated in block **914.** At decision block **916**, the profile analysis module of the profile matching module determines whether one or both users has verified health information (i.e., by a health care provider. If both users have verified health information ("yes" response from the decision block **916**), the application user interface annotates that the users' health information was verified as indicated in block **918**. If one or both users have unverified health information ("no" response from the decision block **916**), the application user interface annotates that the users' health information was unverified as indicated in block **920**. If the user's health information match criteria and the potential partner's health information does not match ("no" response from the decision block **908**), the code generator of the profile matching module generates a non-matching code that correspond with the color red for display on the application user interface as indicated in block **922**. In this regard, the application only shows the users' health status, or whether the two users match or not. Thus, the application safeguards the users' actual medical information and allows the users to maintain privacy. Additionally, the colors (e.g., red, yellow, green, etc.) provide a visual indicator that makes it easy to understand the match result.

In one embodiment, the present system comprises a computer system having a memory unit with instructions stored thereon, and a processor that is configured to execute the instructions, resulting in a downloadable or a non-downloadable application that enables a user to connect with one or more other users for determining whether their health status match each other's health criteria. More specifically, the application is configured to allow the user to automatically or manually input his or her health information (e.g., date of last STD screening, STD screening results) and set one or more filters that define health criteria that the user seeks in their partner. In some embodiments, the filters include, without limitation, the partner's test results, the number of other partners the partner has had (other than the user) since the last checkup, and the period of time since the last checkup, and the like. Based on the user's health information and the filters, the application is configured to determine whether the user's health criteria and the other user's health information is a match or a not a match, and vice versa. Some embodiments of the program are configured to provide a graphic user interface (GUI) for displaying match results. Preferably, the GUI includes colored screens, wherein each of the colors represents different match and/or non-match combinations. For example, a green screen signifies that the user's health criteria and the other user's health information is a match. Similarly, a yellow screen signifies that the user's health criteria and the other user's health information is a match, but one or both of the user's health information is outdated. Optionally, the green screen and yellow screen may comprise additional identifiers for showing that one or both of the user's health information has been verified or unverified by a health care provider. Finally, a red screen signifies that the user's health criteria and the other user's health information is not a match.

Some embodiments of the invention further comprise a security feature, wherein both users must consent to sharing information with each other. More particularly, one of the users can send the other user an invitation to exchange information by text message, email, or in-app messaging, wherein the content of the text message, email, or in-app messaging can comprise a one-time use code or URL that can be activated to give consent in order to determine whether their health criteria and health information match or does not match. Some embodiments may further comprise means for exchanging information via NFC such that users in close proximity can easily give consent and share information.

Some embodiments of the invention comprise a verification feature, wherein the application is configured to automatically generate and transmit signup emails to the user's health care provider via API, wherein the emails comprise a one-time use URL or a token that can be activated by the health care provider to view, enter, and/or verify the user's health information. If the user's health information is outdated, some embodiments of the application may be configured to enable the user to request and confirm a doctor's appointment with the health care provider.

Some embodiments of the invention comprise a method comprising the steps of automatically generating, via the processor, a token or an encrypted code, wherein the token or the code corresponds to the user's health status, further wherein the token or the code is used to determine a match between two or more users. Once the token or the code is used to determine a match, the token or the code expires so that the user's health status is not saved. In this regard, the present method safeguards the user's actual medical data while enabling the user to share the user's health status to prevent spreading of diseases.

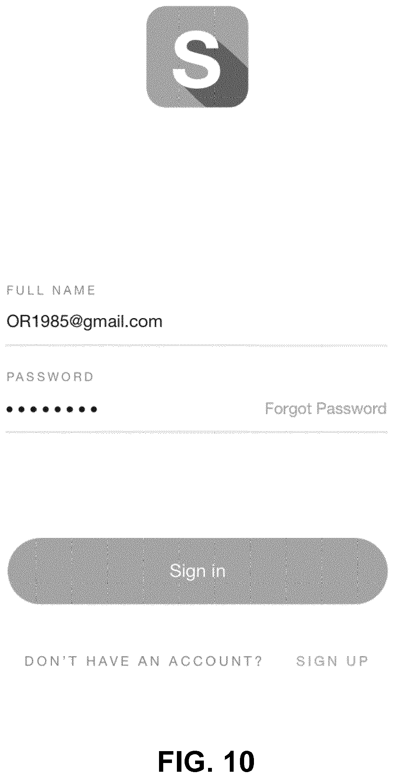

FIGs. 10 through 25 show exemplary GUI of the present application.

Some embodiments of the present system comprise one or more user devices in a network (e.g., the Internet), wherein the user devices comprise various types of computer systems (e.g., a mobile phone, a tablet computer, a personal digital assistant (PDA), an e-reader, etc.) having a memory unit having instructions stored thereon, and a processor that is configured to execute the instructions, resulting in a mobile application. In other embodiments, the system comprises a web application, a website, or other types of downloadable and/or non-downloadable software program.

Referring now to FIG. 10, there is shown an exemplary GUI of the sign-in screen of the application. In operation, a user can sign up and log in, for example, by providing his or her email address, user name, or other types of identifying information. Alternatively, the user can sign up by linking the user's third party social networking accounts (e.g., Facebook®). The user can set up his or her account by providing account related information such as name, location, age, gender, and the like. Account related information that is associated with each of the registered users is stored in a database.

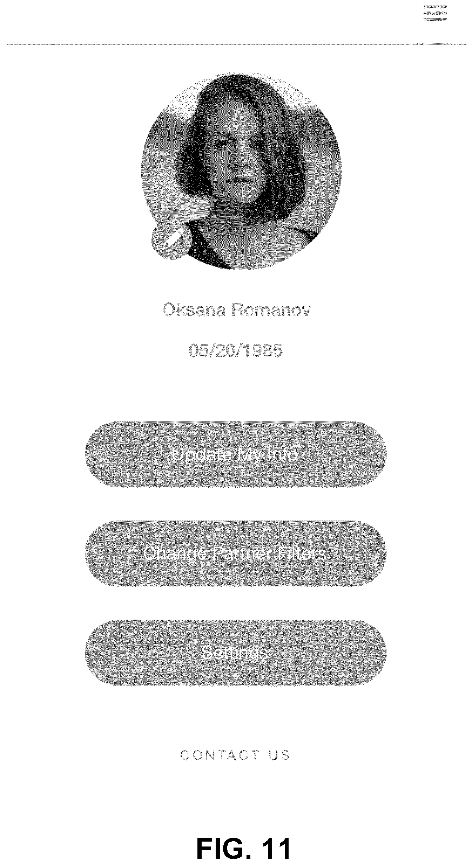

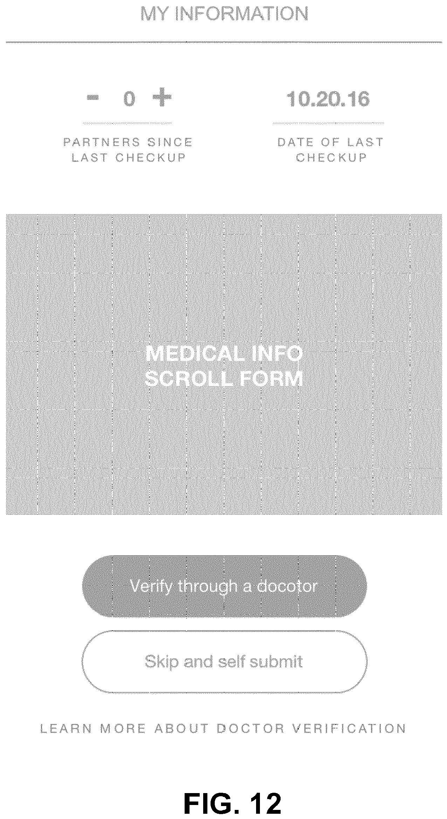

Referring now to FIGs. 11 and 12, there is shown an exemplary user's summary page of the application and an exemplary user information page of the application, respectively. The summary page comprises icons for the updating a user's information, partner filters, and settings. In some embodiments, the home page includes an option for viewing test match, a countdown of the number of days until next doctor's appointment, a link to make a doctor's appointment, and a link to find a match.

In the user's information, the user can provide a photo or an avatar, the user's date of birth, gender, number of partners since last checkup, and a link to verify health information via a doctor or enter health information manually. If the user elects to have his or her information verified by a doctor, the user can activate the button to request his or her health care provider to view, enter, and/or verify the user's health information.

Upon activation, the application automatically transmits a message or an email to a health care provider (i.e., the user's primary care physician) of the user's choice using API, wherein the message or the email comprises a token or a link that enables the health care provider to create a provider account in order to view, enter, and/or confirm the user's STD screening information. In this regard, the provider may be prompted to provide verification or credentialing information to ensure that the provider is qualified to view, enter, and/or confirm the user's health information. In some embodiments, the application may be configured to automatically extract data relating to the user's health information from a database associated with the health care provider.

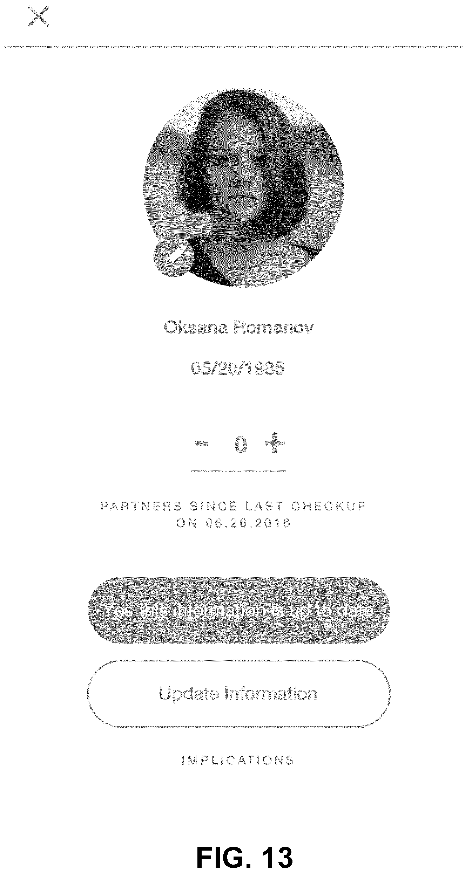

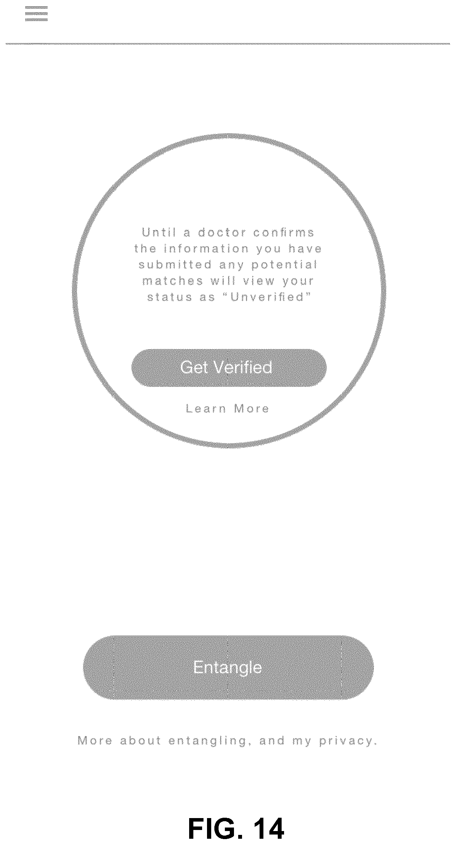

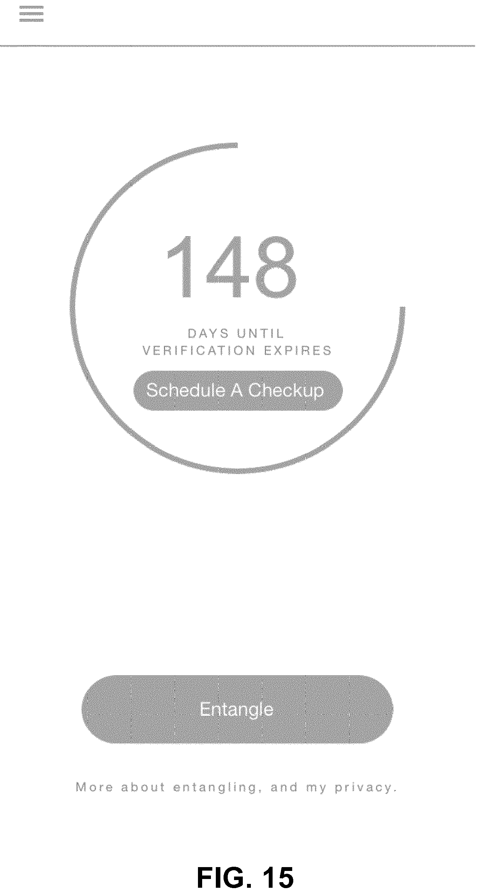

Alternatively, the user can volunteer his or her STD screening information in the GUI. For instance, the user can enter the date that the user was last tested for STDs, and then indicate whether each of the corresponding test yielded a positive or a negative result. For any unknown results, inconclusive results, or for tests that were not conducted, the user can indicate that the result is unknown. Thereafter, the user can confirm that the information is up to date or further update the information, as depicted in FIG. 13. Optionally, users may request their health care provider to verify their health information, as shown in FIG. 14. Upon transmitting a request, the application is configured to relay the users' requests as discussed above. The date that the user was last tested for STDs is used to calculate the number of days until the verification expires, as shown in FIG. 15. The user can schedule a checkup via the application before or after the verification expires.

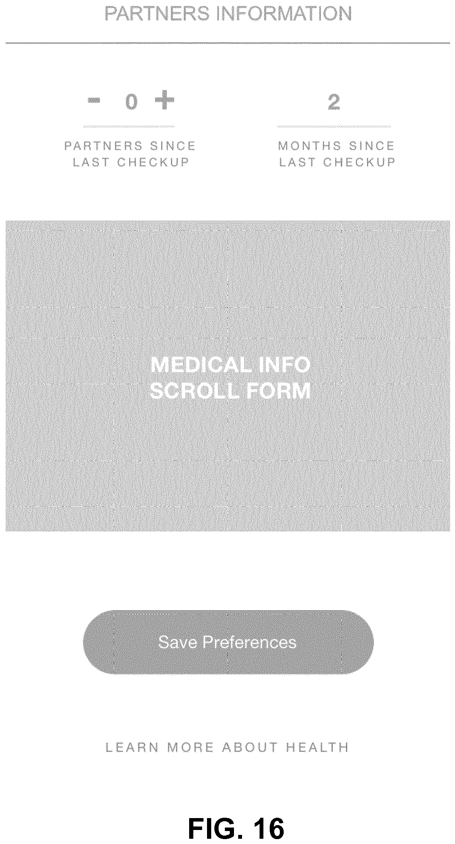

Once the user has provided his or her STD screening information, the user can adjust the partner filters as shown in FIG. 16, wherein the filters indicate the health criteria that the user seeks in a potential sexual partner (i.e., another user of the application). In the illustrated embodiment, the GUI for the filters include the number of partners since the potential partner's last checkup, the number of months since last checkup, and the test results of each STD. The user can adjust filters as needed and save the preferences.

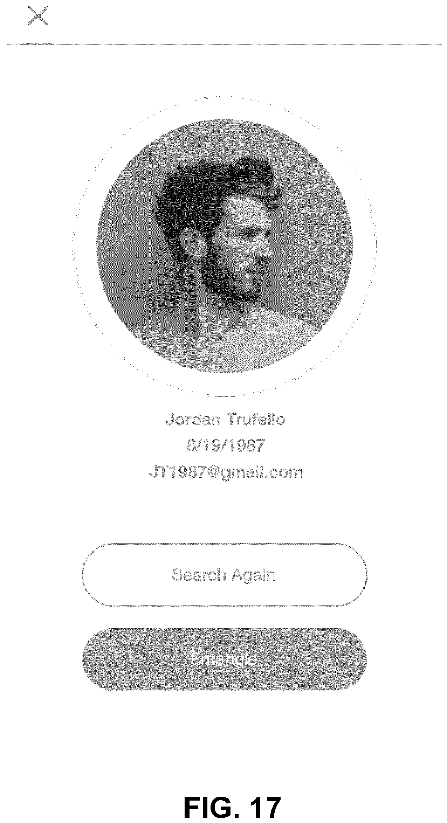

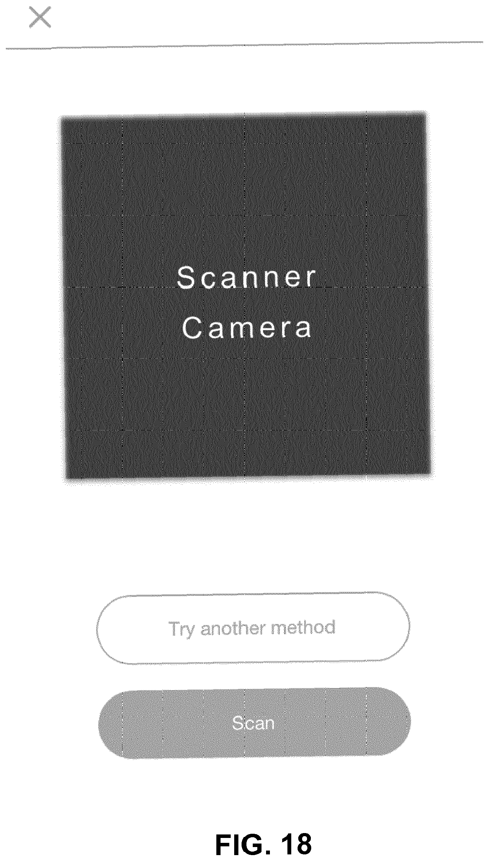

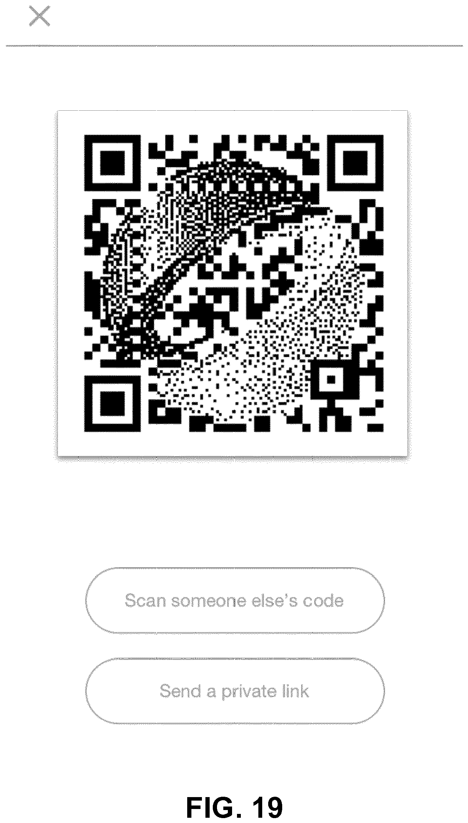

The saved filters create health criteria that is used to determine whether
the user is a match with another user. As shown in FIG. 17, the application is configured to locate another user in order to determine a match. Users can manually locate another user, for example, by entering another user's name or identifier. Alternatively, the application is configured to operate the device's camera to scan for codes associated with another user, as depicted in FIGs. 18 and 19.

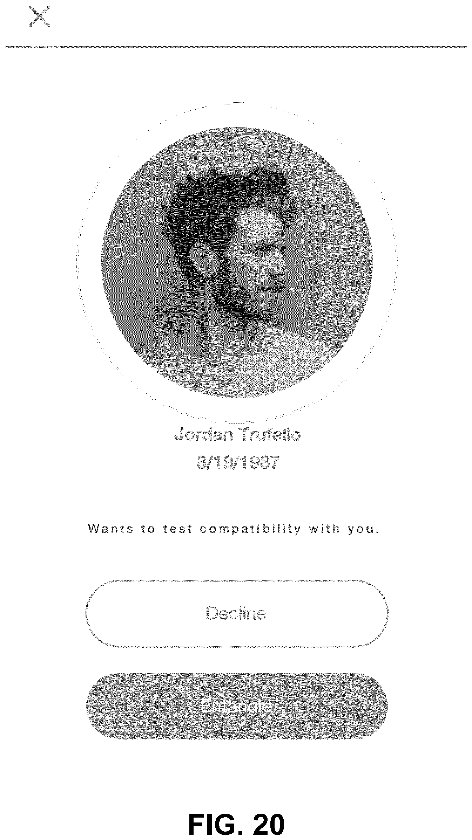

In some embodiments, the application is configured to request the user for consent to share the user's health status, and not the actual medical data, with another user, as shown in FIG. 20. In this regard, one or more users can request the other user for the other user's health status, via the application, by sending a text message, an email, or an in-app message, wherein the content of the text message, email, or the in-app message includes a token or a one- time use code that can be activated by the receiving user to give consent.

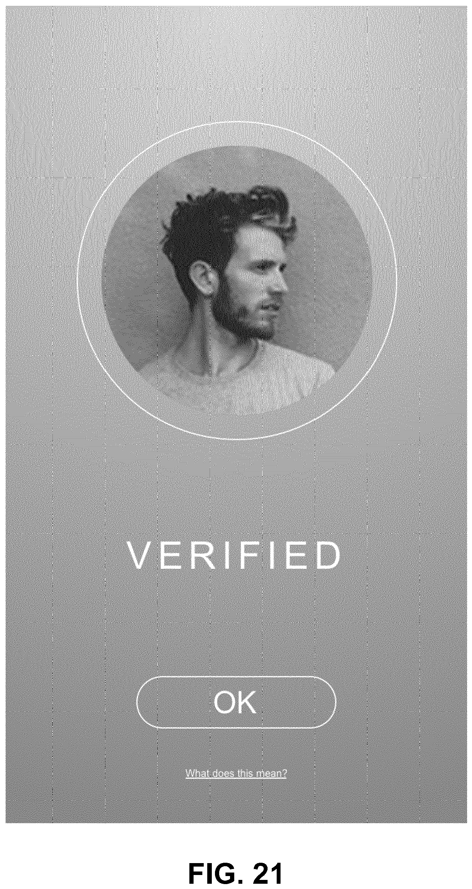

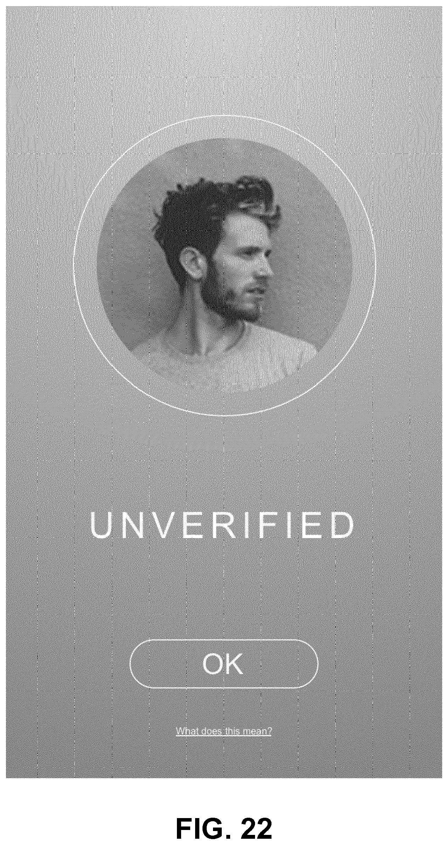

Upon receiving or giving consent, the user can test match with another user. If both of the user's health information match with each other's health criteria, a green screen is displayed. If one or both of the user's health information have been verified by a health care provider, the screen further includes an annotation indicating that the information has been verified, as shown in FIG. 21. If the users' health information has not been verified, the screen indicates that the health information has not been verified, as indicated in FIG. 22.

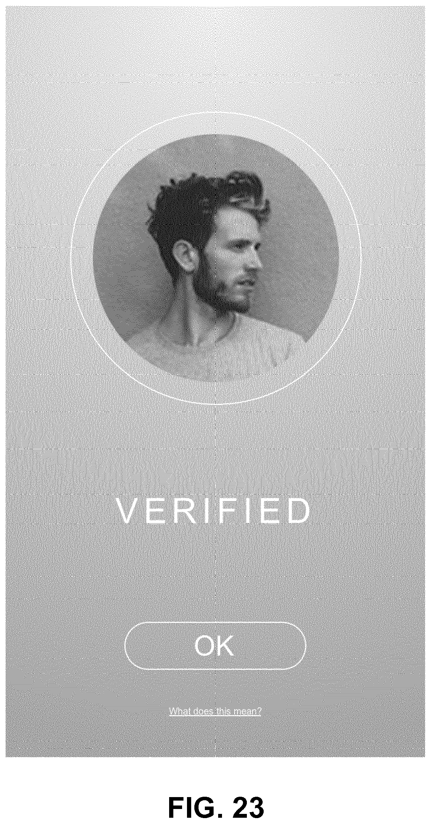

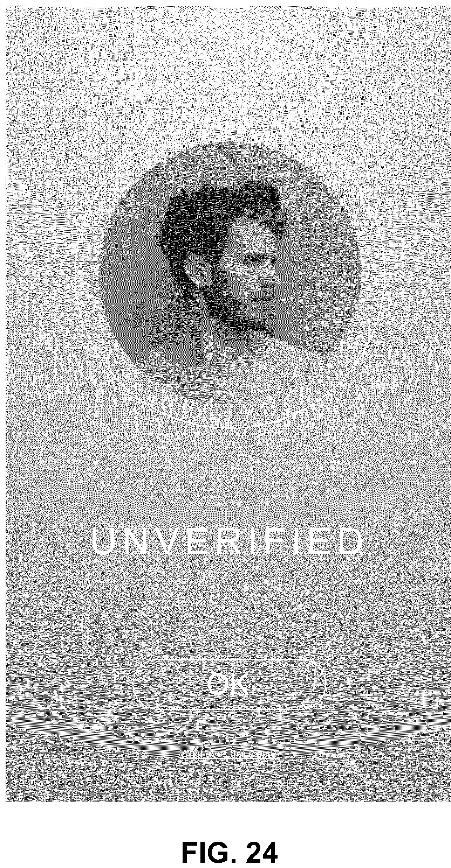

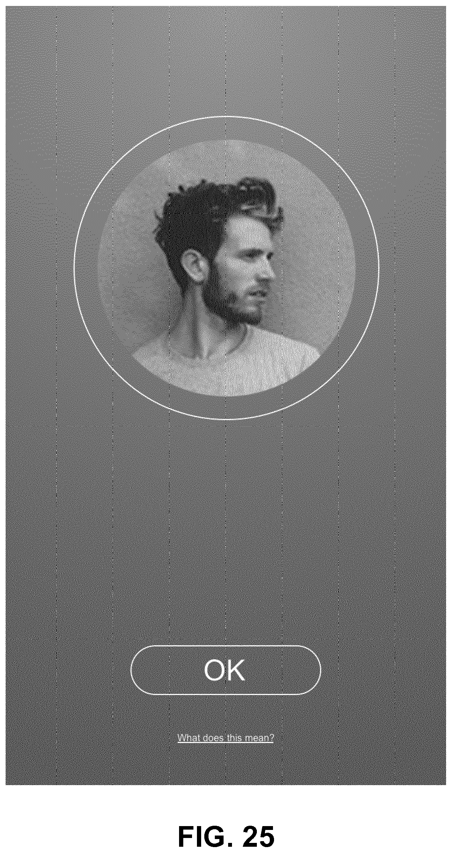

If the users' health information match each other's health criteria, but one or both of the user's health information is out-of-date (i.e., more than a predetermined amount of time has passed since the date of last screening or testing), a yellow screen is displayed. Similar to the green screen, the yellow screen may also indicate that the users' health information has been verified or not verified as shown in FIGs. 23 and 24. If the users' health information and health criteria do not match, a red screen is displayed, as shown in FIG. 25. In this regard, the application only shows the users' health status, or whether the two users match or not. Thus, the application safeguards the users' actual medical information and allows the users to maintain privacy.

In a typical embodiment, a computer-implemented method for displaying on a mobile device health status matches between two or more users, comprises the steps of: receiving health information from a first user and a second user via a user interface, wherein said health information is used to generate a health status; receiving health criteria filters from said first user and said second user; receiving a request from said first user to identify a health status match, wherein said second user has agreed to provide said health status of said second user to said first user; generating a first token associated with said health information of said first user and a second token associated with said health information of said second user; determining whether said health status of said first user match said health criteria of said second user and whether said health status of said second user match said health criteria of said first user based on said first token and said second token; and displaying a match result via said user interface. In a typical embodiment, said health information comprises user's STD test results. In a typical embodiment, the method further comprises the steps of: requesting consent from said first user to provide said health status of said first user to said second user; and generating a third token and transmitting said third token from said second user to said first user to enable said first user to activate said third token. In a typical embodiment, the method further comprises the steps of: requesting verification of said health information from a health care provider; generating a fourth token and transmitting said fourth token from said first user to said health care provider; and verifying said health information of said first user. In a typical embodiment, said health information comprises a date of last checkup; if more than a predetermined amount of time has passed since said date of last checkup, enabling said first user to request a doctor's appointment. In a typical embodiment, the step of displaying said match result comprises the steps of: displaying a green screen with a verified notation if said health status of said first user match said health criteria of said second user and said health status of said second user match said health criteria of said first user and said health information of each of said first user and said second user are verified by a health care provider. In a typical embodiment, the step of displaying said match result comprises the steps of: displaying a green screen with an unverified notation if said health status of said first user match said health criteria of said second user and said health status of said second user match said health criteria of said first user and said health information of each of said first user and said second user are not verified by a health care provider. In a typical embodiment, the step of displaying said match result comprises the steps of: displaying a yellow screen if said health status of said first user match said health criteria of said second user and said health status of said second user match said health criteria of said first user and at least one of said health information of said first user and said second user is out of date. In a typical embodiment, the step of displaying said match result comprises the steps of: displaying a red screen if said health status of said first user does not match said health criteria of said second user or said health status of said second user does not match said health criteria of said first user. In a typical embodiment, a network computer system for displaying health status matches between two or more users comprises: a memory unit for storing instructions and a processor that executes said instructions to enable actions, including: receiving health information from a first user and a second user via a user interface, wherein said health information is used to generate a health status; receiving health criteria filters from said first user and said second user; receiving a request from said first user to identify a health status match, wherein said second user has agreed to provide said health status of said second user to said first user; generating a first token associated with said health information of said first user and a second token associated with said health information of said second user; determining whether said health status of said first user match said health criteria of said second user and whether said health status of said second user match said health criteria of said first user based on said first token and said second token; and displaying a match result via said user interface. In a typical embodiment, said health information comprises user's STD test results. In a typical embodiment, said processor is further configured to: request consent from said first user to provide said health status of said first user to said second user; and generate a third token and transmitting said third token from said second user to said first user to enable said first user to activate said third token. In a typical embodiment, said processor is further configured to: request verification of said health information from a health care provider; generate a fourth token and transmitting said fourth token from said first user to said health care provider; and verify said health information of said first user. In a typical embodiment, said health information comprises a date of last checkup; if more than a predetermined amount of time has passed since said date of last checkup, enabling said first user to request a doctor's appointment. In a typical embodiment, the step of displaying said match result comprises the steps of: displaying a green screen with a verified notation if said health status of said first user match said health criteria of said second user and said health status of said second user match said health criteria of said first user and said health information of each of said first user and said second user are verified by a health care provider. In a typical embodiment, the step of displaying said match result comprises the steps of: displaying a green screen with an unverified notation if said health status of said first user match said health criteria of said second user and said health status of said second user match said health criteria of said first user and said health information of each of said first user and said second user are not verified by a health care provider. In a typical embodiment, the step of displaying said match result comprises the steps of: displaying a yellow screen if said health status of said first user match said health criteria of said second user and said health status of said second user match said health criteria of said first user and at least one of said health information of said first user and said second user is out of date. In a typical embodiment, the step of displaying said match result comprises the steps of: displaying a red screen if said health status of said first user does not match said health criteria of said second user or said health status of said second user does not match said health criteria of said first user.

The foregoing descriptions of specific embodiments of the present invention have been presented for purposes of illustration and description. They are not intended to be exhaustive or to limit the present invention to the precise forms disclosed, and obviously many modifications and variations are possible in light of the above teaching. The exemplary embodiment was chosen and described in order to best explain the principles of the present invention and its practical application, to thereby enable others skilled in the art to best utilize the present invention and various embodiments with various modifications as are suited to the particular use contemplated.

It is therefore submitted that the instant invention has been shown and described in what is considered to be the most practical and preferred embodiments. It is recognized, however, that departures may be made within the scope of the invention and that obvious modifications will occur to a person skilled in the art. With respect to the above description then, it is to be realized that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

## Claims

1. A computer-implemented method for displaying on a mobile device health status matches between two or more users, comprising the steps of:
receiving health information associated with a first user via an application user interface of a match making application, the health information comprising health status of the first user and being received from the first user via a first user device;
receiving health criteria for a potential partner from a second user via the application user interface, the health criteria for the potential partner being received from the second user via a second user device;
generating a health status code associated with the health information of the first user;
determining whether the health status of the first user match the health criteria for the potential partner received from the second user; and
generating a matching code that corresponds with a color for display on the application user interface on the first user device and the second user device.

2. The method of claim 1, wherein said health information comprises user's STD test results.

3. The method according to any of the previous claims, further comprising the steps of:
receiving a request from the first user for verification of the health information of the first user;
generating an access token to transmit to a provider terminal, the provider terminal being operated by a health care provider;
receiving verification of the health information of the first user the verification being received from the health care provider having an access to a patient medical information database comprising the health information of the first user.

4. The method according to any of the previous claims, further comprising the steps of:
receiving, from the first user device, a request to connect the first user device and the second user device;
generating a one-time access code to transmit to the second user device;
authenticating and authorizing the second user device to determine whether the health status of the first user matches the health criteria for the potential partner received from the second user.

5. The method according to any of the previous claims, further comprising the steps of:
receiving, from the first user device, a request to connect the first user device and the second user device;
generating a QR code to display on the application user interface at the first user device;
authenticating and authorizing the second user device to determine whether the health status of the first user matches the health criteria for the potential partner received from the second user.

6. The method according to any of the previous claims, further comprising the steps of:
receiving, from the first user device, a request to connect the first user device and the second user device;
displaying a profile picture of the second user on the application user interface at the first user device;
authenticating and authorizing the second user device to determine whether the health status of the first user matches the health criteria for the potential partner received from the second user.

7. The method according to any of the previous claims, further comprising the steps of:
displaying a prompt to schedule a doctor's appointment on the application user interface;
receiving a request to book the doctor's appointment;
retrieving available dates and times for the doctor's appointment, the available dates and times being received from a health care provider at a provider terminal;
booking the doctor's appointment based at least in part on the selection of one of the available dates and times.

8. The method according to any of the previous claims, wherein the color is green if the health status of the first user matches the health criteria for the potential partner received from the second user and the health status of the first user is current.

9. The method according to any of the previous claims, wherein the color is yellow if the health status of the first user matches the health criteria for the potential partner received from the second user and the health status of the first user is out of date.

10. The method according to any of the previous claims, wherein the color is red if the health status of the first user does not match the health criteria for the potential partner received from the second user.

11. A network computer system for displaying health status matches between two or more users, comprising:
a memory unit for storing instructions and a processor that executes said instructions to enable actions, including:
receiving health information associated with a first user via an application user interface of a match making application, the health information comprising health status of the first user and being received from the first user via a first user device;
receiving health criteria for a potential partner from a second user via the application user interface, the health criteria for the potential partner being received from the second user via a second user device;
generating a health status code associated with the health information of the first user;
determining whether the health status of the first user match the health criteria for the potential partner received from the second user; and
generating a matching code that corresponds with a color for display on the application user interface on the first user device and the second user device.

12. The system of claim 11, wherein said health information comprises user's STD test results.

13. The system according to any of the claims 11 to 12, further comprising the steps of:
receiving a request from the first user for verification of the health information of the first user;
generating an access token to transmit to a provider terminal, the provider terminal being operated by a health care provider;
receiving verification of the health information of the first user the verification being received from the health care provider having an access to a patient medical information database comprising the health information of the first user.

14. The system according to any of the claims 11 to 13, further comprising the steps of:
receiving, from the first user device, a request to connect the first user device and the second user device;
generating a one-time access code to transmit to the second user device;
authenticating and authorizing the second user device to determine whether the health status of the first user matches the health criteria for the potential partner received from the second user.

15. The system according to any of the claims 11 to 14, further comprising the steps of:
receiving, from the first user device, a request to connect the first user device and the second user device;
generating a QR code to display on the application user interface at the first user device;
authenticating and authorizing the second user device to determine whether the health status of the first user matches the health criteria for the potential partner received from the second user.

16. The system according to any of the claims 11 to 15, further comprising the steps of:
receiving, from the first user device, a request to connect the first user device and the second user device;
displaying a profile picture of the second user on the application user interface at the first user device;
authenticating and authorizing the second user device to determine whether the health status of the first user matches the health criteria for the potential partner received from the second user.

17. The system according to any of the claims 11 to 16, further comprising the steps of:
displaying a prompt to schedule a doctor's appointment on the application user interface;
receiving a request to book the doctor's appointment;
retrieving available dates and times for the doctor's appointment, the available dates and times being received from a health care provider at a provider terminal;
booking the doctor's appointment based at least in part on the selection of one of the available dates and times.

18. The system according to any of the claims 11 to 17, wherein the color is green if the health status of the first user matches the health criteria for the potential partner received from the second user and the health status of the first user is current.

19. The system according to any of the claims 11 to 18, wherein the color is yellow if the health status of the first user matches the health criteria for the potential partner received from the second user and the health status of the first user is out of date.

20. The system according to any of the claims 11 to 19, wherein the color is red if the health status of the first user does not match the health criteria for the potential partner received from the second user.
